# EUROPÄISCHE PATENTSCHRIFT

(11) **EP 3 424 901 B1**
(45) Veröffentlichungstag und Bekanntmachung des Hinweises auf die Patenterteilung: **10.06.2020**
(21) Anmeldenummer: 17179827.5
(22) Anmeldetag: 05.07.2017
(51) Int. Cl.: C07C 67/303, C07B 35/04

(54) **HYDRIERUNG VON AROMATISCHEN VERBINDUNGEN**
HYDROGENATION OF AROMATIC COMPOUNDS
HYDROGÉNATION DE COMPOSÉS AROMATIQUES

(43) Veröffentlichungstag der Anmeldung: 09.01.2019
(73) Patentinhaber: BASF SE, 67056 Ludwigshafen am Rhein (DE)
(72) Erfinder: POPLOW, Frank, 67056 Ludwigshafen (DE)
(74) Vertreter: BASF IP Association

(56) Entgegenhaltungen:
- DE-A1- 10 036 172

## Beschreibung

Die vorliegende Erfindung betrifft ein Verfahren zur Hydrierung von aromatischen Verbindungen an einem festen Katalysator, insbesondere ein Verfahren zur Herstellung von alicyclischen Carbonsäureestern durch Hydrierung der entsprechenden aromatischen Carbonsäureester an einem festen Katalysator.

Verfahren zur Hydrierung von aromatischen Verbindungen sind in der chemischen Industrie von zentraler Bedeutung. Sie dienen beispielsweise der Herstellung von alicyclischen Carbonsäuren oder alicyclischen Carbonsäureestern durch Hydrierung von aromatischen Carbonsäuren oder aromatischen Carbonsäureestern.

Alicyclische Carbonsäureester, wie beispielsweise die Ester der Cyclohexan-1,2-dicarbonsäure, werden als Bestandteil von Schmiermitteln, Klebstoffen, Farben oder Lacken, oder als Hilfsmittel bei der Metallverarbeitung eingesetzt. Weiterhin finden sie als Weichmacher für Polyolefine oder PVC Verwendung.

Verfahren zur Hydrierung von aromatischen Carbonsäureestern zu den entsprechenden alicylischen Carbonsäureestern sind dem Fachmann aus dem Stand der Technik bekannt.

So beschreibt beispielsweise die DE 102 32 868 A1 oder die DE 102 25 565 A1 die Hydrierung von aromatischen Polycarbonsäureestern in zwei hintereinander geschalteten Reaktoren, wobei der erste Reaktor in Schlaufenfahrweise (teilweise Rückführung des Reaktoraustrags) und der zweite Reaktor im geraden Durchgang betrieben wird. Die Hydrierung wird dabei in der Flüssig/Gas-Mischphase oder Flüssigphase durchgeführt.

Der vorliegenden Erfindung lag die Aufgabe zugrunde, ein verbessertes Verfahren zur Hydrierung von aromatischen Verbindungen, insbesondere von aromatischen Carbonsäureestern, zur Verfügung zu stellen. Durch das verbesserte Verfahren sollte es möglich sein, den Katalysator besser auszunutzen. Eine verbesserte Katalysatorausnutzung zeigt sich beispielsweise darin, dass bei gleicher Hydriertemperatur ein höherer Hydrierumsatz erzielt wird, oder, für einen bestimmten Hydrierumsatz eine geringere Hydriertemperatur notwendig ist. Dadurch, dass ein bestimmter Hydrierumsatz bei geringerer Hydriertemperatur erzielt werden kann, wird beispielsweise die Lebenszeit des Katalysators erhöht, womit längere Betriebsintervalle möglich sein sollten. Auch sollte eine geringere Hydriertemperatur zu einer geringeren Nebenproduktbildung beitragen. Zudem sollte es durch das erfindungsgemäße Verfahren möglich sein, den der Hydrierung zugeführten Wasserstoff besser zu nutzen, womit Verluste an, zur Hydrierung ungenutztem, Wasserstoff reduziert werden sollen.

Die Aufgabe wird gelöst durch ein Verfahren zur Hydrierung von aromatischen Verbindungen an einem festen Katalysator in Gegenwart eines wasserstoffhaltigen Gases umfassend, einen ersten Reaktor der in Schlaufenfahrweise betrieben wird,
einen zweiten Reaktor der im geraden Durchlauf betrieben wird,
wobei zumindest ein Teil des Austrags des ersten Reaktors dem zweiten Reaktor als Zulauf zugeführt wird, das dadurch gekennzeichnet ist,
dass der erste Reaktor als Rieselbettreaktor ausgestaltet ist und in Rieselbettfahrweise betrieben wird,
und dass der zweite Reaktor derart betrieben wird, dass der darin enthaltene Katalysator teilweise geflutet ist.

In dem erfindungsgemäßen Verfahren wird die Hydrierung der aromatischen Verbindungen kontinuierlich oder diskontinuierlich durchgeführt. Es ist bevorzugt, dass die Hydrierung kontinuierlich durchgeführt wird.

Aromatische Verbindungen werden in dem erfindungsgemäßen Verfahren zu ihren alicyclischen Verbindungen hydriert. Aromatische Verbindungen die in dem erfindungsgemäßen Verfahren eingesetzt werden können, sind beispielsweise Phenole, aromatische Carbonsäuren oder aromatische Carbonsäureester. Aromatische Carbonsäuren oder aromatische Carbonsäureester sind bevorzugt. Aromatische Carbonsäureester sind besonders bevorzugt.

Aromatische Carbonsäuren können eine oder mehrere Carbonsäuregruppen aufweisen. So weisen aromatische Carbonsäuren beispielsweise ein, zwei oder drei Carbonsäuregruppen auf. Die Carbonsäuregruppen sind über ihren Carbonylkohlenstoff mit einem aromatischen Ring verbunden. Aromatische Carbonsäuren weisen einen oder mehrere aromatische Ringe auf. Bei mehreren aromatischen Ringen können die aromatischen Ringe beispielsweise annelliert und/oder verbrückt sein. Weist die aromatische Carbonsäure mehrere aromatische Ringe und mehrere Carbonsäuregruppen auf, können sich die Carbonsäuregruppen an einem aromatischen Ring oder an verschiedenen aromatischen Ringen befinden.

Aromatische Carbonsäuren, die eine Carbonsäuregruppe aufweisen werden als aromatische Monocarbonsäuren bezeichnet. Aromatische Monocarbonsäuren sind beispielsweise Benzoesäure oder 1-Naphthoesäure, wobei Benzoesäure bevorzugt ist.

Aromatische Carbonsäuren, die zwei Carbonsäuregruppen aufweisen werden als aromatische Dicarbonsäuren bezeichnet. Aromatische Dicarbonsäuren sind beispielsweise Phthalsäure, Isophthalsäure, Terephthalsäure, 1,2-Napthalindicarbonsäure, 1,3-Naphthalindicarbonsäure, 1,4-Naphthalindicarbonsäure, 1,5-Napthalindicarbonsäure, 1,7-Naphthalindicarbonsäure oder 1,8-Napthalindicarbonsäure, wobei Phthalsäure, Isophthalsäure oder Terephthalsäure bevorzugt sind.

Aromatische Carbonsäuren, die mehr als zwei Carbonsäuregruppen aufweisen werden als aromatische Polycarbonsäuren bezeichnet. Aromatische Polycarbonsäuren sind beispielsweise Hemimellitäure, Trimellitsäure, Trimesinsäure oder Benzol-1,2,3,4-tetracarbonsäure, wobei Trimellitsäure bevorzugt ist.

Aromatische Carbonsäureester werden beispielsweise durch Veresterung von aromatischen Carbonsäuren mit Alkoholen oder durch Umesterung von aromatischen Carbonsäureestern erhalten. Weist eine aromatische Carbonsäure mehr als eine Carbonsäuregruppe auf, ist es in der Regel bevorzugt, möglichst jede Carbonsäuregruppe zu verestern. Die Carbonsäuregruppen können dabei unabhängig voneinander mit dem gleichen oder mit unterschiedlichen Alkoholen verestert werden. In der Regel ist es bevorzugt, dass die Carbonsäuregruppen mit dem gleichen Alkohol oder mit einer Mischung von Isomeren eines Alkohols verestert werden.

Veresterungsreaktionen und Umesterungsreaktionen sind dem Fachmann bekannt (siehe beispielsweise WO 2015/082676).

Aufgrund einer nicht vollständig ablaufenden Veresterung kann es sein, dass zumindest ein Teil der aromatischen Carbonsäuregruppen nicht verestert wird. Somit können bei der Veresterung auch Mischungen von aromatischen Carbonsäureestern mit unterschiedlichem Veresterungsgrad entstehen. So können bei der Veresterung abhängig von den Ausgangsverbindungen beispielsweise beliebige Mischungen von aromatischen Monocarbonsäureestern, aromatischen Dicarbonsäureestern und aromatischen Polycarbonsäureestern entstehen.

Bei einem Alkohol handelt es sich um eine einzelne chemische Verbindung oder um eine Mischung von Isomeren. Ein Alkohol kann eine oder mehrere Hydroxygruppen aufweisen. Ein Alkohol, der eine Hydroxygruppe aufweist wird als Monool bezeichnet. Ein Alkohol, der zwei Hydroxygruppen aufweist wird als Diol bezeichnet. Ein Alkohol, der mehr als zwei Hydroxygruppen aufweist wird als Polyol bezeichnet.

Ein Alkohol weist bevorzugt 1 bis 18, weiter bevorzugt 3 bis 13, besonders bevorzugt 8 bis 13 und ganz besonders bevorzugt 9 Kohlenstoffatome auf.

Ein Alkohol ist beispielsweise linear oder verzweigt. Bei einem Alkohol handelt es sich beispielsweise um einen Alkyl-, Cycloalkyl- oder Alkoxyalkylalkohol. Es ist bevorzugt, dass es sich bei einem Alkohol um einen Alkylalkohol handelt. Es ist weiter bevorzugt, dass es sich bei einem Alkohol um ein Alkylmonool handelt.

Bei einem Alkylmonool mit 8 bis 13 Kohlenstoffatomen handelt es sich beispielsweise um n-Octanol, iso-Octoanl, 2-Ethylhexanol, n-Nonanol, 2-Propylhexanol, iso-Nonanol, n-Decanol, iso-Decanol, 2-Propylheptanol, n-Undecanol, iso-Undecanol, n-Dodecanol, iso-Dodecanol, n-Tridecanol oder iso-Tridecanol. Alkylmonoole mit 9 Kohlenstoffatomen sind bevorzugt. Iso-Nonanol ist besonders bevorzugt.

Bei iso-Octanol, iso-Nonanol, iso-Decanol, iso-Undecanol, iso-Dodecanol oder iso-Tridecanol handelt es sich um keine Einzelverbindung, sondern um eine Mischung von verschiedenen Isomeren. Die genaue Zusammensetzung einer solchen Isomerenmischung hängt von den Herstellungsbedingungen und/oder den eingesetzten Ausgangsverbindungen ab. Abhängig von den Herstellungsbedingungen und/oder den eingesetzten Ausgangsverbindungen sind in der WO 2015/082676 beispielhaft Zusammensetzungen möglicher Isomerenmischungen offenbart.

Die oben genannten Alkohole beziehungsweise Isomerengemsiche können nach dem Fachmann bekannten Methoden hergestellt werden (siehe beispielsweise Ullmann's Encyclopedia of Industrial Chemistry, 2013 Wiley-VCH, doi: 10.1002/14356007.a01_279.pub2 oder WO 2015/082676).

Aromatische Carbonsäureester, die eine Estergruppe aufweisen werden als aromatische Monocarbonsäureester bezeichnet. Aromatische Monocarbonsäureester sind beispielsweise Phthalsäuremonomethylester, Phthalsäuremonoglykolester, Terephthalsäuremonomethylester, Terephthalsäuremonoglykolester, Isophthalsäuremonomethylester oder Isophthalsäuremonoglykolester.

Aromatische Monocarbonsäureester sind beispielsweise auch Ester der Benzoesäure. Benzoesäure kann dabei mit Monoolen, Diolen oder Polyolen verestert sein. Ester der Benzoesäure mit Monoolen sind beispielsweise iso-Octylbenzoat, 2-Ethylhexylbenzoat, iso-Nonylbenzoat, iso-Decylbenzoat oder 2-Propylheptylbenoat. Ester der Benzoesäure mit Diolen sind beispielsweise Glycoldibenzoat, Diethylenglycoldibenzoat, Triethylenglykoldibenzoat oder Propylenglycoldibenzoat.

Unter den aromatischen Monocarbonsäureestern sind iso-Nonylbenzoat, iso-Decylbenzoat oder 2-Propylheptylbenzoat bevorzugt. Iso-Nonylbenzoat oder iso-Decylbenzoat sind besonders bevorzugt.

Aromatische Carbonsäureester, die zwei Estergruppen aufweisen werden als aromatische Dicarbonsäureester bezeichnet. Aromatische Dicarbonsäureester sind beispielsweise Phthalsäurediester, Isophthalsäurediester, Terephthalsäurediester, 1,2-Napthalindicarbonsäurediester, 1,3-Naphthalindicarbonsäurediester, 1,4-Naphthalindicarbonsäurediester, 1,5-Napthalindicarbonsäurediester, 1,7-Naphthalindicarbonsäurediester oder 1,8-Napthalindicarbonsäurediester, wobei Phthalsäurediester oder Terephthalsäurediester bevorzugt sind. Phthalsäurediester sind besonders bevorzugt.

Phthalsäurediester sind beispielsweise, Phthalsäuredimethylester, Phthalsäurediethylester, Phthalsäuredi-n-propylester, Phthalsäuredi-iso-propylester, Phthalsäuredi-n-butylester, Phthalsäuredi-iso-butylester, Phthalsäuredi-tert-butylester, Phthalsäurediglykolester, Phthalsäure-n-octylester, Phthalsäure-iso-octylester, Phthalsäuredi-2-ethylhexylester, Phthalsäuredi-n-nonylester, Ptthalsäuredi-iso-nonylester. Phthalsäuredi-n-decylester, Phthalsäuredi-iso-decylester, Phthalsäuredi-2-propylheptylester Phthalsäuredi-n-undecylester, Phthalsäuredi-iso-undecylester, Phthalsäuredi-n-dodecylester oder Phthalsäuredi-iso-dodecylester. Phthalsäuredi-iso-nonylester, Phthalsäuredi-iso-decyl oder Phthalsäuredi-2-propylheptylester sind bevorzugt. Phthalsäuredi-iso-nonylester oder Phthalsäuredi-2-propylheptylester sind weiter bevorzugt. Phthalsäuredi-iso-nonylester ist besonders bevorzugt.

Terephthalsäurediester sind beispielsweise Terephthalsäuredimethylester, Terephthalsäurediethylester, Terephthalsäuredi-n-propylester, Terephthalsäuredi-iso-propylester, Terephthalsäuredi-n-butylester, Terephthalsäuredi-iso-butylester,Terephthalsäuredi-tert-butylester, Terephthalsäurediglykolester, Terephthalsäure-n-octylester, Terephthalsäure-iso-octylester, Terepthalsäuredi-2-ethylhexylester, Terephthalsäuredi-n-nonylester, Terepthalsäuredi-iso-nonylester, Terephthalsäuredi-n-decylester, Terephthalsäuredi-iso-decylester, Terephthalsäuredi-2-propylheptylester, Terephthalsäuredi-n-undecylester, Terephthalsäuredi-iso-undecylester, Terephthalsäuredi-n-dodecylester oder Terephthalsäuredi-iso-dodecylester. Terepthalsäuredi-2-ethylhexylester ist bevorzugt.

Unter den aromatischen Dicarbonsäureester sind Phthalsäuredi-iso-nonylester, Phthalsäurediiso-decyl, Phthalsäuredi-2-propylheptylester oder Terepthalsäuredi-2-ethylhexylester bevorzugt. Phthalsäuredi-iso-nonylester oder Terepthalsäuredi-2-ethylhexylester sind weiter bevorzugt. Phthalsäuredi-iso-nonylester ist besonders bevorzugt.

Aromatische Carbonsäureester, die mehr als zwei Estergruppen aufweisen werden auch als aromatische Polycarbonsäureester bezeichnet. Aromatische Polycarbonsäureester werden beispielsweise durch Veresterung von Hemimellitäure, Trimellitsäure, Trimesinsäure oder Benzol-1,2,3,4-tetracarbonsäure mit einem Alkohol oder einer Mischung von Alkoholen erhalten.

Die Hauptprodukte, die durch das erfindungsgemäße Verfahren durch Hydrierung der aromatischen Verbindungen erhalten werden, hängen beispielsweise von den Hydrierbedingungen, wie Temperatur und/oder Druck und/oder dem eingesetzten Katalysator ab. Die Hauptprodukte der Hydrierung werden im Rahmen der vorliegenden Erfindung auch als Hydrierprodukte bezeichnet.

Im Rahmen der vorliegenden Erfindung werden bei der Hydrierung von aromatischen Verbindungen die alicyclischen Verbindungen als Hydrierprodukte erhalten. Handelt es sich bei den aromatischen Verbindungen um aromatische Carbonsäuren werden als Hydrierprodukte die alicyclische Carbonsäuren erhalten. Handelt es sich bei den aromatischen Verbindungen um aromatische Carbonsäureester werden als Hydrierprodukte die alicyclische Carbonsäureester erhalten.

So kann durch das erfindungsgemäße Verfahren beispielsweise aus Phthalsäuredi-iso-nonylester Di-(isononyl)-1,2-cyclohexandicarboxylat als Hydrierprodukt, aus Phthalsäuredi-2-propylheptylester Di-(2-propylheptyl)-1,2-cyclohexandicarboxylat als Hydrierprodukt, aus Phthalsäuredi-iso-decylphthalat Di-2(isodecyl)-1,2-cyclohexandicarboxalt oder aus Terepthalsäuredi-2-ethylhexylester Di-(2-ethylhexyl)-1,4-cyclohexandicarboxylat als Hydrierprodukt erhalten werden.

In dem erfindungsgemäßen Verfahren werden die aromatischen Verbindungen an einem festen Katalysator in Gegenwart eines wasserstoffhaltigen Gases hydriert. Es ist bevorzugt, dass die aromatischen Verbindungen an einem festen Katalysator in Gegenwart eines wasserstoffhaltigen Gases kontinuierlich hydriert werden.

Es ist bevorzugt, dass der Katalysator im Festbett als regellose Schüttung oder als Packung im ersten und zweiten Reaktor angeordnet ist. Es ist weiter bevorzugt, dass der Katalysator im Festbett als regellose Schüttung im ersten und zweiten Reaktor angeordnet ist. Ein Reaktor kann dabei ein oder mehrere in Reihe geschaltete Katalysatorbetten enthalten. Bei mehreren in Reihe geschalteten Katalysatorbetten kann die Länge der Katalysatorbetten variieren.

Es ist bevorzugt, dass das Katalysatorvolumen in einem Verhältnis von 85:15 bis 60:40 zwischen dem ersten und zweiten Reaktor aufgeteilt ist.

Zur Hydrierung der aromatischen Verbindungen, vorzugsweise zur Hydrierung von aromatischen Carbonsäuren oder aromatischen Carbonsäureestern zu den entsprechenden alicyclischen Carbonsäuren oder alicyclischen Carbonsäureestern enthält der Katalysator bevorzugt Metalle und/oder Metalloxide der VI. bis VIII. Nebengruppe des Periodensystems der Elemente, insbesondere Platin, Rhodium, Palladium, Kobalt, Nickel und/oder Ruthenium als katalytisch aktive Komponente, wobei Ruthenium besonders bevorzugt ist. Ein solcher Katalysator kann zudem Metalle und/oder Metalloxide der V. oder IX. Nebengruppe des Periodensystems der Elemente, insbesondere Rhenium und/oder Kupfer enthalten. Die Metalle und/oder Metalloxide können auf einem Trägermaterial abgeschieden sein. Sind die Metalle und/oder Metalloxide auf einem Trägermaterial abgeschieden, wird bei der Herstellung eines solchen Katalysators in der Regel ein Trägermaterial mit einem mittleren Porendurchmesser, der im Bereich von 2 bis 50 nm liegt, verwendet (die Bestimmung des mittleren Porendurchmessers erfolgt durch HG-Porosimetrie, insbesondere nach der DIN 66133). Das Trägermaterial kann Aktivkohle, Siliciumcarbid, Aluminiumoxid, Siliziumoxid, Alumosilikat, Titandioxid, Zirkondioxid, Magnesiumoxid, Zinkoxid oder ein Gemisch aus zwei oder mehr der genannten Komponenten enthalten. Das Trägermaterial kann zudem Alkalimetalle, Alkalimetalloxide, Erdalkalimetalle, Erdalkalimetalloxide und/oder Schwefel enthalten. Katalysatoren, die sich bevorzugt für die Hydrierung von aromatischen Verbindungen, insbesondere aromatischen Carbonsäuren oder aromatischen Carbonsäureestern eigenen sind beispielsweise in der EP-B1 1042273B1, DE-A 10232868, DE-A 10225565 oder EP-A2 1676828 beschrieben. Ein bevorzugter Katalysator weist beispielsweise einen Ru-Gehalt von 0,45 bis 0,55 Gewichtsprozent bezogen auf das Gewicht des Trägermaterials, eine spezifische Oberfläche von 220 bis 290 m²/g (BET, ISO 9277) und ein Porenvolumen 0,48 bis 0,62 ml/g (Hg-Porosimetrie, DIN 66133) auf, das Trägermaterial umfasst Aluminiumoxid. Ein weiterer, bevorzugter Katalysator weist beispielsweise einen Ru-Gehalt von 0,3 bis 0,45 Gewichtsprozent bezogen auf das Gewicht des Trägermaterials, eine spezifische Oberfläche von 310 bis 360 m²/g (BET, ISO 9277) und ein Porenvolumen von 0,7 bis 0,9 ml/g (Hg-Porosimetrie, DIN 66133) auf, das Trägermaterial umfasst Siliziumoxid.

Bei einem wasserstoffhaltigen Gas handelt es sich um beliebige wasserstoffhaltige Gasgemische, die keine schädlichen Mengen an Katalysatorgiften wie Kohlenstoffmonoxid und/oder Schwefelverbindungen enthalten. Ein wasserstoffhaltiges Gas kann ein Inertmedium enthalten. Ein Inertmedium geht unter den Reaktionsbedingungen keine Reaktion mit den Edukten, Produkten und dem Katalysator ein. Bei einem Inertmedium kann es sich um Stickstoff, Methan, Helium, Argon, oder um eine Mischung dieser handeln. Es ist bevorzugt, dass als wasserstoffhaltiges Gas möglichst reiner Wasserstoff eingesetzt wird. Möglichst reiner Wasserstoff weist eine Reinheit von mindestens 90 Prozent, bevorzugt mindestens 95 Prozent und besonders bevorzugt mindestens 98 Prozent auf. Die Verwendung von möglichst reinem Wasserstoff hat den Vorteil, dass die Aufpegelung des Inertmedium im Reaktionsraum reduziert wird. Eine Aufpegelung des Inertmediums im Reaktionsraum würde zur einer Reduktion des Wasserstoffpartialdrucks führen, was sich negativ auf die Raum-Zeit-Ausbeute der Hydrierungsreaktion auswirken würde.

Gemäß dem erfindungsgemäßen Verfahren können aromatische Verbindungen, vorzugsweise aromatische Carbonsäureester, an einem festen Katalysator und in Gegenwart eines wasserstoffhaltigen Gases mit einem Wasserstoffanteil von mindestens 95 Prozent und bevorzugt mindestens 98 Prozent, kontinuierlich hydriert werden.

Die Menge an Wasserstoff, die in dem erfindungsgemäßen Verfahren eingesetzt wird entspricht mindestens der stöchiometrischen Menge, die zur Hydrierung der aromatischen Verbindungen benötigt wird. Es ist jedoch bevorzugt einen Überschuss von bis zu 30 Prozent über der zur Hydrierung stöchiometrisch benötigten Menge einzusetzen. Es ist weiter bevorzugt, dass ein Überschuss von bis zu 20 Prozent und weiter bevorzugt ein Überschuss von bis zu 10 Prozent über der stöchiometrisch benötigten Menge eingesetzt wird.

Das erfindungsgemäße Verfahren zur Hydrierung der aromatischen Verbindungen umfasst einen ersten Reaktor, der in Schlaufenfahrweise und einen zweiten Reaktor der im geraden Durchlauf betrieben wird. Der zweite Reaktor ist dabei derart mit dem ersten Reaktor verbunden, dass zumindest ein Teil des Austrags des ersten Reaktors dem zweiten Reaktor zugeführt wird. Es ist bevorzugt, dass es sich bei dem ersten und bei dem zweiten Reaktor um einen Rohrreaktor, insbesondere um einen vertikalen Rohrreaktor handelt. Ebenfalls ist es bevorzugt, dass der erste und der zweite Reaktor keine Heiz- oder Kühlvorrichtungen aufweisen.

Anstelle des ersten Reaktors, der in Schlaufenfahrweise betrieben wird, können auch mehrere Reaktoren, die in Schlaufenfahrweise betrieben werden und in Reihe oder parallel geschaltet sind, eingesetzt werden. Anstelle des zweiten Reaktors, der im geraden Durchlauf betrieben wird, können auch mehrere Reaktoren, die im geraden Durchlauf betrieben werden und in Reihe oder parallel geschaltet sind, eingesetzt werden. Es ist jedoch bevorzugt, dass nur ein Reaktor in Schlaufenfahrweise und nur ein Reaktor im geraden Durchlauf eingesetzt werden.

Jeder der Reaktoren kann ein oder mehrere Katalysatorbetten enthalten, wobei die Katalysatorbetten in Reihe geschaltet sind. Die Katalysatorbetten können in ihrer Länge variieren.

Der erste Reaktor in Schlaufenfahrweise ist als Rieselbettreaktor ausgestaltet und wird in Rieselbettfahrweise betrieben. Auch wenn es nicht bevorzugt ist, ist es generell möglich, den ersten Reaktor als Schlaufenreaktor in Sumpffahrweise zu betreiben.

Als Zulauf werden dem ersten Reaktor Kreisstrom und Frischzulauf zugeführt. Hierzu kann der Kreisstrom beispielsweise mit dem Frischzulauf vermischt und die Mischung dem ersten Reaktor zugeführt werden. Kreisstrom und Frischzulauf können dem ersten Reaktor aber auch separat zugeführt werden. Der Frischzulauf enthält aromatische Verbindungen, die im Rahmen des erfindungsgemäßen Verfahren hydriert werden sollen. Die Flüssigkeitsbelastung des Katalysators mit Frischzulauf beträgt bevorzugt 50 bis 1000 kg_{Frischzulauf}/(Stunde ^{∗} m³_{Gesamtkatalysatorvolumen}). Bei dem Gesamtkatalysatorvolumen handelt es sich um das Katalysatorvolumen, das über die eingesetzten Reaktoren verteilt ist. Es ist weiter bevorzugt, dass die Flüssigkeitsbelastung des Katalysators mit Frischzulauf 100 bis 500 kg_{Frischzulauf}/(Stunde ^{∗} m³_{Gesamtkatalysatorvolumen}) beträgt.

Der Zulauf weist bevorzugt eine Temperatur von 70 bis 150°C und weiter bevorzugt eine Temperatur von 80 bis 120°C auf, wenn dieser dem ersten Reaktor zugeführt wird. So weist der Zulauf beispielsweise eine Temperatur von 85, 90, 95, 100, 105, 110 oder 115°C auf. Werden Kreisstrom und Frischzulauf dem ersten Reaktor separat zugeführt, weisen diese unabhängig voneinander bevorzugt eine Temperatur von 70 bis 150°C und weiter bevorzugt eine Temperatur von 80 bis 120°C auf. So können Kreisstrom und Frischzulauf beispielsweise unabhängig voneinander eine Temperatur von 85, 90, 95, 100, 105, 110 oder 115°C aufweisen.

Der Austrag des ersten Reaktors wird in einen Kreisstrom und in einen Hydrierstrom aufgetrennt. Der Kreisstrom wird in den ersten Reaktor zurückgeführt, der Hydrierstrom wird im zweiten Reaktor der weiteren Hydrierung zugeführt. Die Auftrennung des Austrags aus dem ersten Reaktor in einen Kreisstrom und in einen Hydrierstrom kann nach dem Fachmann bekannten Methoden zur Stromteilung erfolgen, beispielsweise mittels einer Pumpenvorlage mit Überlauf, oder einem Stromteiler. Es ist bevorzugt, dass der Kreisstrom vor Rückführung in den ersten Reaktor gekühlt wird. Der Austrag wird dem ersten Reaktor im unteren Teil, beispielsweise am Boden entnommen.

Das Umlaufverhältnis zwischen Kreisstrom zu Hydrierstrom beträgt bevorzugt 1:1 bis 20:1. Es ist weiter bevorzugt, dass das Umlaufverhältnis 2:1 bis 15:1 beträgt. Es ist besonders bevorzugt, dass das Umlaufverhältnis 4:1 bis 10:1 beträgt. So beträgt das Umlaufverhältnis beispielsweise 5:1, 6:1, 7:1, 8:1 oder 9:1.

Im Austrag des ersten Reaktors beträgt der Hydrierumsatz der aromatischen Verbindungen bevorzugt 80 bis 98 Prozent bezogen auf den Gesamthydrierumsatz im ersten und zweiten Reaktor, weiter bevorzugt 85 bis 95 Prozent. Werden beispielsweise aromatische Carbonsäuren oder aromatische Carbonsäureester als aromatische Verbindungen eingesetzt, beträgt der Hydrierumsatz bevorzugt 80 bis 98 Prozent bezogen auf den Gesamthydrierumsatz im ersten und zweiten Reaktor und weiter bevorzugt 85 bis 95 Prozent. So beträgt der Hydrierumsatz im ersten Reaktor beispielsweise 86, 88, 90, 92 oder 94 Prozent.

Da der Austrag des ersten Reaktors in der Regel eine höhere Temperatur aufweist als der Frischzulauf, wenn dieser dem ersten Reaktor zugeführt wird, kann der Austrag des ersten Reaktors gekühlt werden, bevor dieser in einen Kreisstrom und einen Hydrierstrom aufgetrennt wird. Auch kann der Kreisstrom nach Auftrennung des Austrags aus dem ersten Reaktor gekühlt werden. Die Kühlung des Austrags und/oder des Kreisstroms kann in einem oder mehreren Wärmetauschern erfolgen. Es kann von Vorteil sein, die Kühlung des Austrags und/oder des Kreisstroms mit der Erwärmung des Frischzulaufs wärmezukoppeln.

Das Umlaufverhältnis zwischen Kreisstrom zu Frischzulauf beträgt im ersten Reaktor bevorzugt 1:1 bis 20:1. Es ist weiter bevorzugt, dass das Umlaufverhältnis 2:1 bis 15:1 beträgt. Es ist besonders bevorzugt, dass das Umlaufverhältnis 4:1 bis 10:1 beträgt. So kann das Umlaufverhältnis beispielsweise 5:1, 6:1, 7:1, 8:1 oder 9:1 betragen.

Kreisstrom und Frischzulauf werden dem ersten Reaktor so zugeführt, dass eine möglichst gleichmäßige Verteilung der Flüssigkeiten über den Reaktorquerschnitt gewährleistet ist. Dies hat den Vorteil einer möglichst gleichmäßigen Katalysatorausnutzung. Die Zuführung von Kreisstrom und Frischzulauf kann hierzu über eine oder mehrere, über den Reaktorquerschnitt verteilte, Zuführungen erfolgen. Die Zuführungen befinden sich im oberen Teil des Reaktors, oberhalb des Katalysators. Des Weiteren kann im Reaktor oberhalb des Katalysators und unterhalb der Zuführungen eine oder mehrere Verteilereinrichtungen, wie Verteilerbleche, angeordnet sein.

Die Flüssigkeitsbelastung des Katalysators (Leerrohrgeschwindigkeit) im ersten Reaktor beträgt bevorzugt 30 bis 180 m/h, weiter bevorzugt 50 bis 150 m/h und besonders bevorzugt 60 bis 120 m/h. So beträgt die Leerrohrgeschwindigkeit im ersten Reaktor beispielsweise 70, 80, 85, 90, 95, 100, 105, 110 oder 115 m/h. Eine ausreichend hohe Flüssigkeitsbelastung stellt eine vollständige Benetzung des gesamten Katalysatorbetts sicher. So wird vermieden, dass sich Flüssigkeitssträhnen ausbilden und ein Teil des Katalysators ungenutzt bleibt. Im Übrigen wird der Stoffaustausch umso intensiver, je höher die Flüssigkeitsbelastung ist. Dadurch kann beispielsweise die Raum-Zeit-Ausbeute positiv beeinflusst werden.

Die Hydrierung der aromatischen Verbindungen kann in Abwesenheit oder in Gegenwart eines inerten Lösungsmittels durchgeführt werden. Als inerte Lösungsmittel eignen sich alle Flüssigkeiten, die unter den Reaktionsbedingungen keine Reaktion mit den Edukten, Produkten und dem Katalysator eingehen, die homogene Lösungen mit den Edukten und Produkten bilden und sich von den Produkten leicht abtrennen lassen. Bei dem inerten Lösungsmittel kann es sich auch um eine Mischung von verschiedenen Flüssigkeiten handeln. Bei einem inerten Lösungsmittel kann es sich beispielsweise um das Hydrierprodukt der jeweils ablaufenden Hydrierung, um eine Isomerenmischung von langkettigen Kohlenwasserstoffen, oder um eine Isomerenmischung von Alkoholen mit 9 bis 13 Kohlenstoffatomen handeln.

Der Einsatz eines inerten Lösungsmittels hat beispielsweise den Vorteil, dass sich die Temperaturentwicklung der Hydrierung besser kontrollieren lässt.

Wird die Hydrierung der aromatischen Verbindungen in Gegenwart eines inerten Lösungsmittels durchgeführt kann das inerte Lösungsmittel mit dem Kreisstrom und/oder dem Frischzulauf dem ersten Reaktor zugeführt werden. Das inerte Lösungsmittel kann dabei mit dem Kreisstrom und/oder dem Frischzulauf vermischt werden, bevor die dadurch erhaltene Mischung dem ersten Reaktor zugeführt wird.

Weist der erste Reaktor mehrere in Reihe geschaltete Katalysatorbetten auf kann das inerte Lösungsmittel auch zwischen den Katalysatorbetten dem ersten Reaktor zugeführt werden. Dies kann unabhängig davon erfolgen, ob inertes Lösungsmittel mit dem Kreisstrom und/oder dem Frischzulauf dem ersten Reaktor zugeführt wird. Wird das inerte Lösungsmittel zwischen den Katalysatorbetten dem ersten Reaktor zugeführt kann dieses mit wasserstoffhaltigem Gas vermischt sein.

Das wasserstoffhaltige Gas wird dem ersten Reaktor zugeführt. Das wasserstoffhaltige Gas wird dabei separat oder mit dem Kreisstrom und/oder Frischzulauf vermischt dem ersten Reaktor zugeführt. Es ist bevorzugt, dass das wasserstoffhaltige Gas dem ersten Reaktor separat von Kreisstrom und/oder Frischzulauf zugeführt wird. Wird das wasserstoffhaltige Gas dem ersten Reaktor separat zugeführt kann es über eine oder mehrere, über den Reaktorquerschnitt verteilte, Zuführungen erfolgen. Das wasserstoffhaltige Gas wird dem ersten Reaktor im Gleich- oder Gegenstrom zugeführt, wobei es bevorzugt ist, dass das wasserstoffhaltige Gas dem ersten Reaktor im Gleichstrom zugeführt wird. Wird das wasserstoffhaltige Gas dem ersten Reaktor im Gleichstrom zugeführt, befinden sich eine oder mehrere Zuführungen für das wasserstoffhaltige Gas in der Regel im oberen Teil des Reaktors, oberhalb des Katalysators.

Weist der erste Reaktor mehrere in Reihe geschaltete Katalysatorbetten auf, kann ein Teil des wasserstoffhaltigen Gases auch zwischen den Katalysatorbetten dem ersten Reaktor zugeführt werden. Dies kann den Vorteil haben, dass die Wärmeentwicklung der Hydrierungsreaktion besser kontrolliert werden kann. Das wasserstoffhaltige Gas kann hierzu direkt oder vermischt mit einem inerten Lösungsmittel dem ersten Reaktor zugeführt werden.

Die Hydrierung der aromatischen Verbindungen im ersten Reaktor kann adiabatisch, polytrop oder praktisch isotherm, das heißt mit einem Temperaturanstieg von typischerweise weniger als 10 bis 15 °C ausgeführt werden.

Generell ist es bevorzugt, die Hydrierung der aromatischen Verbindungen im ersten Reaktor weitgehend adiabatisch auszuführen. Die Hydrierung der aromatischen Verbindungen wird dabei so ausgeführt, dass der Austrag beim Austritt aus dem ersten Reaktor bevorzugt eine Temperatur von 90 bis 180°C oder weniger aufweist. Es ist weiter bevorzugt, dass der Austrag beim Austritt aus dem ersten Reaktor eine Temperatur von 90 bis 160°C oder weniger aufweist.

Die Temperatur des Austrags beim Austritt aus dem ersten Reaktors ist beispielsweise von der Temperatur des Frischzulaufs, von der Temperatur des Kreisstroms der dem ersten Reaktor zugeführt wird, vom Hydrierumsatz im ersten Reaktor, vom Verhältnis zwischen Frischzulauf zu Kreisstrom und/oder vom Verhältnis zwischen Hydrierstrom zu Kreisstrom abhängig.

Für die Hydrierung der aromatischen Verbindungen beträgt der Druck im ersten Reaktor bevorzugt 50 bis 500 bar, weiter bevorzugt 100 bis 400 bar und besonders bevorzugt 200 bis 280 bar. So kann der Druck im ersten Reaktor beispielsweise 210, 220, 230, 240, 250, 260 oder 270 bar betragen.

Werden aromatische Carbonsäuren oder aromatische Carbonsäureester als aromatische Verbindungen eingesetzt, beträgt der Druck im ersten Reaktor bevorzugt 50 bis 500 bar, weiter bevorzugt 100 bis 400 bar, besonders bevorzugt 150 bis 300 bar und besonders bevorzugt 200 bis 280 bar. So kann der Druck im ersten Reaktor beispielsweise 210, 220, 230, 240, 250, 260 oder 270 bar betragen.

Der zweite Reaktor wird im geraden Durchlauf betrieben. Der Katalysator im zweiten Reaktor ist teilweise geflutet. Es ist bevorzugt, dass der nicht geflutete Teil des Katalysators im zweiten Reaktor in Rieselfahrweise betrieben wird. Eine Flutung des Katalysators kann durch Anstauung des Zulaufs des zweiten Reaktors und Standhaltung erzeugt werden. Methoden zur Anstauung und Standhaltung von Flüssigkeiten in Reaktoren bzw. in Behältern sind dem Fachmann bekannt. Der Grad der Flutung des Katalysators kann beispielsweise über eine Differenzdruckmessung in Kombination mit einer Regelung des Austragsvolumenstroms aus dem zweiten Reaktor gesteuert werden.

Bei einer teilweisen Flutung des Katalysators ist ein Teil des Katalysatorvolumens von einer kontinuierlichen Flüssigphase umgeben. Bevorzugt ist der Katalysator im zweiten Reaktor derart geflutet, dass 10 bis 98 Prozent des Katalysatorvolumens von einer kontinuierlichen Flüssigkeitsphase umgeben sind. Es ist weiter bevorzugt, dass 15 bis 95 Prozent des Katalysatorvolumens von einer kontinuierlichen Flüssigkeitsphase umgeben sind. Es ist besonders bevorzugt, dass 50 bis 95 Prozent des Katalysatorvolumens von einer kontinuierlichen Flüssigkeitsphase umgeben sind. So können bei einer zumindest teilweisen Flutung des Katalysators beispielsweise 55, 60, 65, 70, 75, 80, 85 oder 90 Prozent des Katalysatorvolumens von einer kontinuierlichen Flüssigkeitsphase umgeben sein.

Der Hydrierstrom bildet den Zulauf des zweiten Reaktors. Der Hydrierstrom wird dem zweiten Reaktor im oberen Teil des Reaktors, oberhalb des Katalysators zugeführt. Es ist bevorzugt, dass der Hydrierstrom dem zweiten Reaktor so zugeführt wird, dass eine möglichst gleichmäßige Verteilung des Hydrierstroms über den Reaktorquerschnitt gewährleistet ist. Dies hat den Vorteil einer möglichst gleichmäßigen Katalysatorausnutzung. Die Zuführung des Hydrierstroms kann über eine oder mehrere, über den Reaktorquerschnitt verteilte, Zuführungen erfolgen. Die Zuführungen befinden sich im oberen Teil des Reaktors, oberhalb des Katalysators. Des Weiteren können im Reaktor oberhalb des Katalysators und unterhalb der Zuführungen eine oder mehrere Verteilereinrichtungen, wie Verteilerbleche, angeordnet sein. Dem zweiten Reaktor wird in der Regel kein frisches wasserstoffhaltiges Gas zugeführt, auch wenn dies generell möglich ist.

Der Hydrierstrom weist bevorzugt eine Temperatur von 80 bis 180°C und weiter bevorzugt eine Temperatur von 90 bis 160°C auf, wenn dieser dem zweiten Reaktor zugeführt wird. So kann der Hydrierstrom beispielsweise eine Temperatur von 95, 105, 110, 115, 120, 125, 130, 135, 140,145, 150 oder 155°C aufweisen, wenn dieser dem zweiten Reaktor zugeführt wird. Um eine entsprechende Temperatur des Hydrierstroms einzustellen kann dieser beispielsweise mittels Wärmetauscher, erwärmt oder gekühlt werden.

In einer bevorzugten Ausführungsform umfasst der zweite Reaktor in Form eines vertikalen Rohrreaktors im oberen Teil einen freien Raum und im unteren Teil ein erstes und ein zweites Kompartiment. Das erste Kompartiment ist als nach oben geöffneter Zylinder ausgestaltet und bildet einen Ringraum aus. Das zweite Kompartiment bildet einen den Ringraum umgebenen Ringspaltraum aus. Das erste Kompartiment ist im Wesentlichen konzentrisch im zweiten Reaktor angeordnet. Die Außenwandung des ersten Kompartiments und die Innenwandung des zweiten Reaktors bilden den Ringspaltraum aus. Der Ringraum und der den Ringraum umgebende Ringspaltraum sind zum oberen Teil des Reaktors hin geöffnet. Der Ringraum und der den Ringraum umgebende Ringspaltraum sind in der Ebene ihrer Querschnitte nicht fluidleitend miteinander verbunden. Der untere Teil des zweiten Reaktors erstreckt sich bevorzugt über die unteren 30 bis 80 Prozent des Innenvolumens des Reaktors. Der obere Teil des zweiten Reaktors erstreckt sich bevorzugt über die oberen 70 bis 20 Prozent des Innenvolumens des Reaktors.

Der Austrag des ersten Reaktors wird dem zweiten Reaktor im oberen Teil des zweiten Reaktors im Bereich des freien Raums zugeführt. Der Austrag des ersten Reaktors wird dem zweiten Reaktor bevorzugt so zugeführt, dass eine möglichst gleichmäßige Verteilung des Austrags des ersten Reaktors über den Reaktorquerschnitt des zweiten Reaktors gewährleistet ist. Die Zuführung des Austrags aus dem ersten Reaktor kann hierzu über eine oder mehrere, über den Reaktorquerschnitt verteilte, Zuführungen erfolgen. Die Zuführungen befinden sich im oberen Teil des Reaktors im Bereich des freien Raums. Ebenfalls im oberen Teil und unterhalb der Zuführungen können eine oder mehrere Verteilereinrichtungen, wie Verteilerbleche, angeordnet sein.

Der Katalysator ist entweder im Ringraum oder im Ringspaltraum angeordnet. Es ist bevorzugt, dass der Katalysator im Ringraum angeordnet ist. Der Ringraum oder der Ringspaltraum kann ein oder mehrere Katalysatorbetten enthalten. Enthält der Ringraum oder Ringspaltraum mehrere Katalysatorbetten sind diese in der Regel in Reihe geschaltet. Die Länge der einzelnen Katalysatorbetten kann variieren.

Das Kompartiment, das den Katalysator enthält wird im geraden Durchlauf betrieben, wobei der Katalysator teilweise geflutet ist. Der Teil des Katalysators der nicht geflutet ist wird bevorzugt in Rieselfahrweise betrieben.

Das Kompartiment, das nicht den Katalysator enthält ist flüssigkeitsleitend mit dem ersten Reaktor verbunden. Der Teil des Zulaufs des zweiten Reaktors, der diesem Kompartiment zugeführt wird, wird zumindest teilweise in den ersten Reaktor rückgeführt und stellt damit den Kreisstrom dar. Dies hat beispielsweise den Vorteil, dass der Austrag aus dem ersten Reaktor dem zweiten Reaktor ohne vorherige Auftrennung in einen Hydrierstrom und in einen Kreisstrom zugeführt werden kann.

So kann es bevorzugt sein, dass der zweite Reaktor in Form eines vertikalen Rohrreaktors ein als im Wesentlichen konzentrisch angeordneter, nach oben geöffneter Zylinder ausgebildetes erstes Kompartiment zur Aufnahme des Katalysators und ein als Ringraum zwischen der Außenwandung des ersten Kompartiments und der Innenwandung des Reaktors ausgebildetes zweites Kompartiment zur Aufnahme eines Flüssigphasenvolumens umfasst. Es ist weiter bevorzugt, dass das zweite Kompartiment flüssigkeitsleitend mit dem ersten Reaktor verbunden ist.

Ist der Katalysator im ersten Kompartiment angeordnet ist es besonders bevorzugt, dass das zweite Kompartiment über einen Überlauf flüssigkeitsdurchgängig mit dem ersten Kompartiment verbunden ist. Der Austrag aus dem ersten Reaktor wird dem zweiten Reaktor dabei so zugeführt, dass der gesamte Zulauf des zweiten Reaktors zuerst dem zweiten Kompartiment zugeführt wird. Dies kann beispielsweise durch eine geeignete Anordnung der Zuführungen und/oder Verteilereinrichtungen erreicht werden. Eine derartige Anordnung hat den Vorteil, dass im zweiten Kompartiment stets ein Flüssigkeitsvolumen vorgehalten wird. Erst beim Erreichen eines bestimmten Flüssigkeitsspiegels tritt Flüssigphase aus dem zweiten Kompartiment in das erste Kompartiment über und durchläuft den darin angeordneten Katalysator. Es ist bevorzugt den Überlauf derart auszugestalten, dass eine möglichst gleichmäßige Verteilung der übertretenden Flüssigkeit über den Katalysator ermöglicht wird. Dies kann beispielsweise durch Verteilereinrichtungen erfolgen, die im ersten Kompartiment angeordnet sind. Eine derartige Ausführungsform des zweiten Reaktors ist beispielsweise in der DE 100 36 172 A1 beschrieben, auf deren Inhalt, betreffend den darin beschriebenen zweiten Hydrierreaktor, in vollen Umfang Bezug genommen wird. Das zweite Kompartiment ist dabei flüssigkeitsleitend mit dem ersten Reaktor verbunden womit zumindest ein Teil des Zulaufs in das zweite Kompartiment als Kreisstrom in den ersten Reaktor rückgeführt wird.

Durch die beschriebene Ausgestaltung des zweiten Reaktors wird der Austrag aus dem ersten Reaktor, der dem zweiten Reaktor zugeführt wird, innerhalb des zweiten Reaktors in einen Kreisstrom und in einen Hydrierstrom getrennt. Der Teil des Austrags des ersten Reaktors, der innerhalb des zweiten Reaktors dem Kompartiment zugeführt wird, das den Katalysator enthält stellt dabei den Hydrierstrom dar. Der Teil des Austrags des ersten Reaktors, der innerhalb des zweiten Reaktors dem Kompartiment entnommen wird, das keinen Katalysator enthält und in den ersten Reaktor rückgeführt wird, stellt den Kreisstrom dar.

Die Flüssigkeitsbelastung des Katalysators (Leerrohrgeschwindigkeit) im zweiten Reaktor beträgt bevorzugt 5 bis 100 m/h, weiter bevorzugt 10 bis 80 m/h und besonders bevorzugt 15 bis 70 m/h. Die Flüssigkeitsbelastung des Katalysators im zweiten Reaktor beträgt beispielsweise 20, 25, 30, 35, 40, 45, 50, 55, 60 oder 65 m/h.

Die Hydrierung der aromatischen Verbindungen im zweiten Reaktor kann adiabatisch, polytrop oder praktisch isotherm, das heißt mit einem Temperaturanstieg von typischerweise weniger als 10 bis 15 °C ausgeführt werden.

Generell ist es bevorzugt die Hydrierung der aromatischen Verbindungen im zweiten Reaktor adiabatisch auszuführen. Die Hydrierung der aromatischen Verbindungen wird dabei so ausgeführt, dass der Austrag beim Austritt aus dem zweiten Reaktor eine Temperatur von 220°C oder weniger aufweist. Es ist bevorzugt, dass der Austrag beim Austritt aus dem zweiten Reaktor eine Temperatur von 180°C oder weniger aufweist. So kann der Austrag beim Austritt aus dem zweiten Reaktor beispielsweise eine Temperatur von 100 bis 220°C aufweisen. Bevorzugt kann der Austrag beim Austritt aus dem zweiten Reaktor eine Temperatur von 100 bis 180°C aufweisen. Der Austrag wird dem zweiten Reaktor im unteren Teil, beispielsweise am Boden entnommen. Weist der zweite Reaktor ein erstes und ein zweites Kompartiment auf, wir der Austrag dem Kompartiment entnommen, das den Katalysator enthält. Der Austrag wird dabei bevorzugt im unteren Teil des entsprechenden Kompartiments entnommen, beispielsweise am Boden.

Für die Hydrierung der aromatischen Verbindungen beträgt der Druck im zweiten Reaktor bevorzugt 50 bis 500 bar. Es ist weiter bevorzugt, dass der Druck im zweiten Reaktor 100 bis 400 bar, besonders bevorzugt 200 bis 280 bar beträgt.

Werden aromatische Carbonsäuren oder aromatische Carbonsäureester als aromatische Verbindungen eingesetzt, beträgt der Druck im zweiten Reaktor bevorzugt 50 bis 500 bar, weiter bevorzugt 100 bis 400 bar und besonders bevorzugt 210 bis 280 bar. So kann der Druck im zweiten Reaktor beispielsweise 220, 230, 240, 250, 260 oder 270 bar betragen.

Generell ist es bevorzugt, dass der erste und der zweite Reaktor über eine gemeinsame Druckhaltung verfügen und demnach in beiden Reaktoren vergleichbare Drücke vorliegen. Bei vergleichbaren Drücken unterscheiden sich die Drücke im ersten und zweiten Reaktor maximal um 10 bar, bevorzugt um maximal 2 bar.

Der Hydrierumsatz im zweiten Reaktor beträgt bevorzugt 2 bis 20 Prozent bezogen auf den Gesamthydrierumsatz im ersten und zweiten Reaktor, weiter bevorzugt 5 bis 15 Prozent. Werden beispielsweise aromatische Carbonsäuren oder aromatische Carbonsäureester als aromatische Verbindungen eingesetzt, beträgt der Hydrierumsatz im zweiten Reaktor bevorzugt 2 bis 20 Prozent bezogen auf den Gesamthydrierumsatz im ersten und zweiten Reaktor, weiter bevorzugt 5 bis 15 Prozent.

Im Austrag des zweiten Reaktors beträgt der Hydrierumsatz der aromatischen Verbindungen bevorzugt mindestens 95 Prozent, weiter bevorzugt mindestens 98 Prozent und besonders bevorzugt mindestens 99,9 Prozent, bezogen auf den Gesamthydrierumsatz im ersten und zweiten Reaktor. So beträgt der Hydrierumsatz bevorzugt 95 bis 100 Prozent, weiter bevorzugt 98 bis 100 Prozent und besonders bevorzugt 99,9 bis 100 Prozent. Der Hydrierumsatz der aromatischen Verbindungen im Austrag des zweiten Reaktors beträgt beispielsweise 99,99 Prozent oder mehr.

Werden in dem erfindungsgemäßen Verfahren aromatische Carbonsäuren oder aromatische Carbonsäureester, wie Phthalsäuredi-iso-nonylester oder Terepthalsäuredi-2-ethylhexylester, als aromatische Verbindungen eingesetzt beträgt der Restaromatengehalt bezogen auf die Hydrierprodukte im Austrag des zweiten Reaktors bevorzugt maximal 100 ppm, weiter bevorzugt maximal 50 ppm.

Der Austrag aus dem zweiten Reaktor kann nach dem Fachmann bekannten Methoden aufgearbeitet werden, um die Hydrierprodukte zu isolieren. So kann der Austrag aus dem zweiten Reaktor beispielsweise einer Destillation und/oder Strippung unterzogen werden, um die Hydrierprodukte zu isolieren.

Eine Destillation kann als einfache Destillation oder Rektifikation ausgeführt werden. Eine Destillation kann in einer oder mehreren, in Reihe oder parallel geschalteten, Kolonnen ausgeführt werden. Als Kolonnen für die Destillation eignen sich beispielsweise Bodenkolonnen, wie Ventilbodenkolonne. Kolonnen mit Packungen sind bevorzugt. Bei Packungen handelt es sich beispielsweise um regellose Schüttungen oder um geordnete Packungen. Geordnete Packungen sind bevorzugt. Die Zahl der Trennstufen kann der Fachmann aufgrund seines Fachwissens und durch wenige Routineversuche an die gewünschte Trennwirkung anpassen.

Bei einer Strippung wird der Austrag im Gleich- oder Gegenstrom mit einem Strippmedium in Kontakt gebracht. Bei einem Strippmedium handelt es sich beispielsweise um Gase wie Wasserdampf, Stickstoff und/oder Wasserstoff. Eine Strippung kann in einer oder mehreren, in Reihe oder parallel geschalteten, Kolonnen ausgeführt werden. Als Kolonnen für die Strippung eigenen sich beispielsweise Bodenkolonnen, wie Ventilbodenkolonnen. Kolonnen mit Packungen sind bevorzugt. Bei Packungen handelt es sich beispielsweise um regellose Schüttungen oder um geordnete Packungen. Geordnete Packungen sind bevorzugt.

Handelt es sich bei den Hydrierprodukten um alicyclische Carbonsäureerster, wie Di-(isononyl)-1,2-cyclohexandicarboxylat, Di-(2-propylheptyl)-1,2-cyclohexandicarboxylat, Di-2(isodecyl)-1,2-cyclohexandicarboxalt oder Di-(2-ethylhexyl)-1,4-cyclohexandicarboxylat, können diese beispielsweise als Weichmacher oder als Bestandteil von Weichmacherzusammensetzungen für Kunststoffe wie PVC verwendet werden. Eine weitere Verwendung dieser Verbindungen stellt beispielsweise die Herstellung von Plastisolen, insbesondere PVC-Plastisolen dar.

Bei der Verwendung als Weichmacher oder in der Herstellung von Plastisolen kann es von Vorteil sein, dass die genannten Verbindungen zusammen mit anderen Weichmachern, vorteilhafterweise Weichmacher, die die Geliertemperatur herabsetzen, eingesetzt werden.

### Vorteile erfindungsgemäßes Verfahren

Ein Vorteil des erfindungsgemäßen Verfahrens liegt beispielsweise in einer besseren Katalysatorausnutzung. Eine bessere Katalysatorausnutzung kann sich beispielsweise darin zeigen, dass bei gleicher Hydriertemperatur ein höherer Hydrierumsatz erzielt wird, oder bei einem bestimmten Hydrierumsatz eine geringer Hydriertemperatur benötigt wird. Eine niedrigere Hydriertemperatur hat beispielsweise eine geringere Nebenproduktbildung zum Vorteil. Auch führt eine niedrigere Hydriertemperatur im Allgemeinen zu einer höheren Lebenszeit des Katalysators, wodurch längere Betriebsintervalle möglich sind.

### Beispiel

Die Beispiele dienen lediglich der Veranschaulichung der Erfindung und sind nicht einschränkend auszulegen.

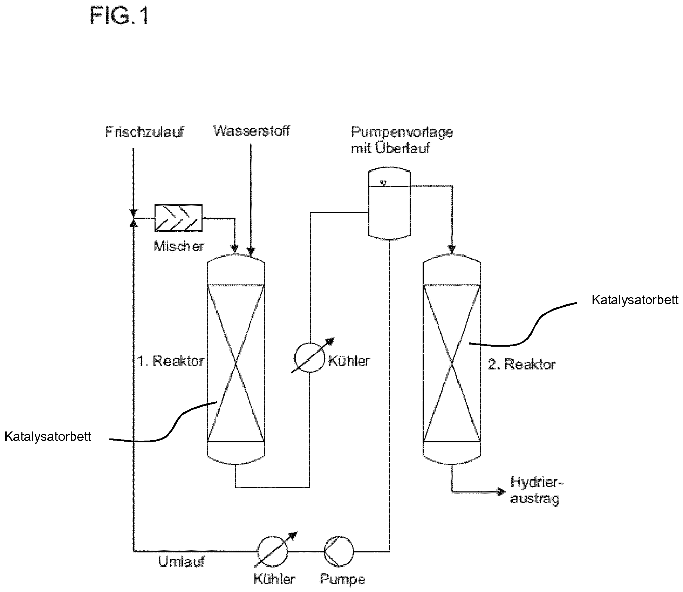

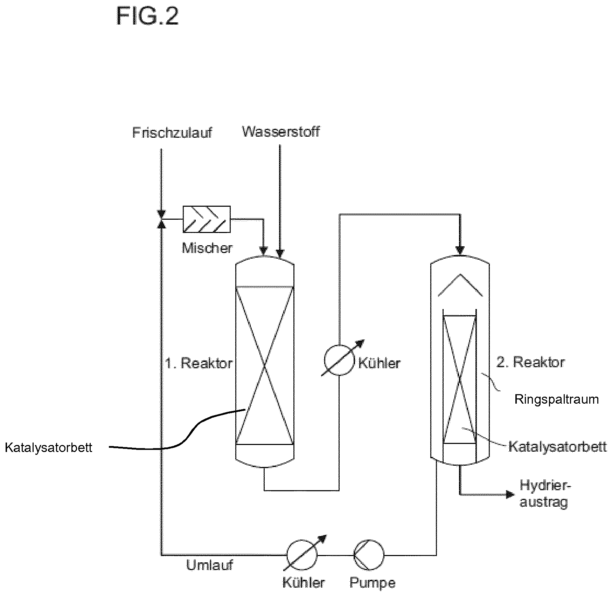

In einer industriellen Hydrieranlage gemäß Figur 1 oder Figur 2 wurde Phthalsäuredi-iso-nonylester zu Di-(isononyl)-1,2-cyclohexandicarboxylat hydriert. Die Hydrierung findet in zwei in Reihe geschalteten Reaktoren statt. Der Katalysator ist im Festbett in beiden Reaktoren angeordnet. 72% des Gesamtkatalysatorvolumens befindet sich im ersten Reaktor, 28% des Gesamtkatalysatorvolumens befindet sich im zweiten Reaktor. Der eingesetzte Katalysator weist einen Rutheniumgehalt von 0,5 Gewichtsprozent bezogen auf das Gesamtgewicht des Trägermateris, eine spezifische Oberfläche von 220 bis 290 m²/g (BET, ISO9277) und ein Porenvolumen von 0,48 bis 0,62 ml/g (Hg-Porosimetrie, DIN 66133) auf, das Trägermaterial umfasst Aluminiumoxid. Der erste Reaktor wird in Schlaufenfahrweise betrieben, der zweite Reaktor wird im geraden Durchlauf betrieben.

Der Wasserstoffdruck betrug in beiden Reaktoren 215 bar. Die Hydrieranlage wurde mit einem Phthalsäuredi-iso-nonylester Frischzulauf von 292 kg/(Stunde ^{∗}m³Gesamtkatalysatorvolumen) betrieben. Das Umlaufverhältnis im ersten Reaktor zwischen Kreisstrom zu Frischzulauf betrug 12:1. Die Hydrieranalage wird derart betrieben, dass im Austrag aus dem zweiten Reaktor der Restaromatengehalt bezogen auf Di-(isononyl)-1,2-cyclohexandicarboxylat unter 100 ppm liegt.

### Vergleichsfahrweise:

Der erste und der zweite Reaktor wurden in Rieselbettfahrweise betrieben. Es wurden folgende Zulauftemperaturen eingestellt:
Erster Reaktor: 97°C
Zweiter Reaktor: 109°C

Die Wasserstoffverluste betrugen bei dieser Fahrweise 3 kg/t Di-(isononyl)-1,2-cyclohexandicarboxylat.

### Erfindungsgemäße Fahrweise:

Der erste Reaktor wurde in Rieselbettfahrweise betrieben. Im zweiten Reaktor war der Katalysator zu 70% geflutet. Es wurden folgende Zulauftemperauren eingestellt:
Erster Reaktor: 92°C
Zweiter Reaktor: 98°C

Die Wasserstoffverluste betrugen bei dieser Fahrweise 0,5 kg/t Di-(isononyl)-1,2-cyclohexandicarboxylat.

### Vergleich der Fahrweisen:

Es werden Betriebsprunkte mit der gleichen Last verglichen. Durch Flutung des Katalysators im zweiten Reaktor wurden folgende Vorteile erzielt:
- Verminderung der Wasserstoffverluste
- Absenkung der Zulauftemperatur des ersten Reaktors um 5°C
- Absenkung der Zulauftemperatur des zweiten Reaktors um 11 °C
Durch eine geringere Zulauftemperatur steigt die Restlebensdauer des Katalysators.

## Patentansprüche

1. Verfahren zur Hydrierung von aromatischen Verbindungen an einem festen Katalysator in Gegenwart eines wasserstoffhaltigen Gases umfassend,
einen ersten Reaktor der in Schlaufenfahrweise betrieben wird,
einen zweiten Reaktor der im geraden Durchlauf betrieben wird,
zumindest ein Teil des Austrags des ersten Reaktors dem zweiten Reaktor zugeführt wird,
**dadurch gekennzeichnet,**
**dass** der erste Reaktor als Rieselbettreaktor ausgestaltet ist und in Rieselbettfahrweise betrieben wird
und der zweite Reaktor derart betrieben wird, dass der darin enthaltene Katalysator teilweise geflutet ist.

2. Verfahren nach Anspruch 1, wobei der nicht geflutete Teil des Katalysators im zweiten Reaktor in Rieselbettfahrweise betrieben wird.

3. Verfahren nach Anspruch 1 oder Anspruch 2, wobei das Umlaufverhältnis im ersten Reaktor zwischen Kreisstrom zu Frischzulauf 1:1 bis 20:1 beträgt.

4. Verfahren einem der Ansprüche 1 bis 3, wobei der Druck im ersten und im zweiten Reaktor 50 bis 500 bar beträgt.

5. Verfahren nach einem der Ansprüche 1 bis 4, wobei die Zulauftemperatur für den ersten Reaktor von 70 bis 150 °C beträgt und die Zulauftemperatur für den zweiten Reaktor von 80 bis 180 °C beträgt.

6. Verfahren nach einem der Ansprüche 1 bis 5, wobei 10 bis 98 Prozent des Katalysatorvolumens im zweiten Reaktor von einer kontinuierlichen Flüssigkeitsphase umgeben sind.

7. Verfahren nach einem der Ansprüche 1 bis 6, wobei es sich bei den aromatischen Verbindungen um aromatische Carbonsäureester handelt und diese zu den entsprechenden alicyclischen Carbonsäureestern hydriert werden.

8. Verfahren nach Anspruch 7, wobei als aromatische Carbonsäureester, Mono- Di-, oder Polycarbonsäureester eingesetzt werden.

9. Verfahren nach einem der Ansprüche 1 bis 6, wobei es sich bei den aromatischen Verbindungen um Phthalsäuredi-iso-nonylester oder um Terepthalsäuredi-2-ethylhexylester handelt.

10. Verfahren nach einem der Ansprüche 1 bis 6, wobei es sich bei den aromatischen Verbindungen um Isononylbenzoat oder um Decylbenzoat handelt.

11. Verfahren nach einem der Ansprüche 1 bis 10, wobei der Hydrierumsatz im zweiten Reaktor 2 bis 20 Prozent bezogen auf den Gesamthydrierumsatz im ersten und zweiten Reaktor beträgt.

12. Verfahren nach einem der Ansprüche 7 bis 10, wobei der Katalysator ein Metall der achten Nebengruppe des Periodensystems der Elemente enthält.

13. Verfahren nach Anspruch 12, wobei der Katalysator Ruthenium enthält.

14. Verfahren nach einem der Ansprüche 1 bis 13, wobei der zweite Reaktor in Form eines vertikalen Rohrreaktors ein als im Wesentlichen konzentrisch angeordneter, nach oben geöffneter Zylinder ausgebildetes erstes Kompartiment zur Aufnahme des Katalysators und ein als Ringraum zwischen der Außenwandung des ersten Kompartiments und der Innenwandung des Reaktors ausgebildetes zweites Kompartiment zur Aufnahme eines Flüssigphasenvolumens umfasst.

15. Verfahren nach einem der Ansprüche 1 bis 14, wobei der Wasserstoffanteil in dem wasserstoffhaltigen Gas mindestens 95 Prozent beträgt.

## Claims

1. A process for hydrogenating aromatic compounds over a solid catalyst in the presence of a hydrogenous gas, comprising
operation of a first reactor in loop mode,
operation of a second reactor in straight pass,
feeding of at least a portion of the output from the first reactor to the second reactor,
which comprises
operating the first reactor in trickle bed reactor configuration and in trickle bed mode
and operating the second reactor in such a way that the catalyst present therein is partly flooded.

2. The process according to claim 1, wherein the unflooded portion of the catalyst in the second reactor is operated in trickle bed mode.

3. The process according to claim 1 or claim 2, wherein the circulation ratio in the first reactor between circulation stream and fresh feed is 1:1 to 20:1.

4. The process according to any of claims 1 to 3, wherein the pressure in the first and second reactors is 50 to 500 bar.

5. The process according to any of claims 1 to 4, wherein the feed temperature for the first reactor is from 70 to 150°C and the feed temperature for the second reactor is from 80 to 180°C.

6. The process according to any of claims 1 to 5, wherein 10 to 98 percent of the catalyst volume in the second reactor is surrounded by a continuous liquid phase.

7. The process according to any of claims 1 to 6, wherein the aromatic compounds are aromatic carboxylic esters and these are hydrogenated to the corresponding alicyclic carboxylic esters.

8. The process according to claim 7, wherein the aromatic carboxylic esters used are mono-, di- or polycarboxylic esters.

9. The process according to any of claims 1 to 6, wherein the aromatic compounds are diisononyl phthalate or di-2-ethylhexyl terephthalate.

10. The process according to any of claims 1 to 6, wherein the aromatic compounds are isononyl benzoate or decyl benzoate.

11. The process according to any of claims 1 to 10, wherein the hydrogenation conversion in the second reactor is 2 to 20 percent based on the overall hydrogenation conversion in the first and second reactors.

12. The process according to any of claims 7 to 10, wherein the catalyst comprises a metal from the eighth transition group of the Periodic Table of the Elements.

13. The process according to claim 12, wherein the catalyst comprises ruthenium.

14. The process according to any of claims 1 to 13, wherein the second reactor, in the form of a vertical tubular reactor, comprises a first compartment in the form of an essentially concentric cylinder open at the top for accommodation of the catalyst and a second compartment in the form of an annular space between the outer wall of the first compartment and the inner wall of the reactor for accommodation of a liquid phase volume.

15. The process according to any of claims 1 to 14, wherein the hydrogen content in the hydrogenous gas is at least 95%.

## Revendications

1. Procédé pour l'hydrogénation de composés aromatiques sur un catalyseur solide en présence d'un gaz contenant de l'hydrogène comprenant
un premier réacteur qui fonctionne en mode boucle,
un deuxième réacteur qui fonctionne en mode passage droit,
au moins une partie de l'évacuation du premier réacteur étant fournie au deuxième réacteur,
**caractérisé en ce que**,
le premier réacteur est agencé comme un réacteur à lit fluidisé et fonctionne en mode de lit fluidisé
et le deuxième réacteur fonctionne de telle manière que le catalyseur contenu dans celui-ci est partiellement inondé.

2. Procédé selon la revendication 1, la partie non inondée du catalyseur dans le deuxième réacteur fonctionnant en mode de lit fluidisé.

3. Procédé selon la revendication 1 ou la revendication 2, le rapport de circulation dans le premier réacteur entre courant circulaire et arrivée de frais est de 1:1 à 20:1.

4. Procédé selon l'une quelconque des revendications 1 à 3, la pression dans le premier et dans le deuxième réacteur étant de 50 à 500 bars.

5. Procédé selon l'une quelconque des revendications 1 à 4, la température d'introduction pour le premier réacteur étant de 70 à 150 °C et la température d'introduction pour le deuxième réacteur étant de 80 à 180 °C.

6. Procédé selon l'une quelconque des revendications 1 à 5, 10 à 98 pour cent du volume de catalyseur dans le deuxième réacteur étant entourés d'une phase liquide continue.

7. Procédé selon l'une quelconque des revendications 1 à 6, les composés aromatiques étant des esters d'acides carboxyliques aromatiques et ceux-ci étant hydrogénés en esters d'acides carboxyliques alicycliques correspondants.

8. Procédé selon la revendication 7, des esters d'acides monocarboxyliques, dicarboxyliques ou polycarboxyliques étant utilisés en tant qu'esters d'acides carboxyliques aromatiques.

9. Procédé selon l'une quelconque des revendications 1 à 6, les composés aromatiques étant l'ester d'isononyle de l'acide phtalique ou l'ester de 2-éthylhexyle de l'acide téréphtalique.

10. Procédé selon l'une quelconque des revendications 1 à 6, les composés aromatiques étant le benzoate d'isononyle ou le benzoate de décyle.

11. Procédé selon l'une quelconque des revendications 1 à 10, la conversion d'hydrogénation dans le deuxième réacteur étant 2 à 20 pour cent par rapport à la conversion d'hydrogénation totale dans le premier et le deuxième réacteur.

12. Procédé selon l'une quelconque des revendications 7 à 10, le catalyseur contenant un métal du huitième sous-groupe du système périodique des éléments.

13. Procédé selon la revendication 12, le catalyseur contenant du ruthénium.

14. Procédé selon l'une quelconque des revendications 1 à 13, le deuxième réacteur sous forme d'un réacteur tubulaire vertical comprenant un premier compartiment formé en cylindres ouverts vers le haut, disposés essentiellement de manière concentrique, pour la réception du catalyseur et un deuxième compartiment formé comme espace annulaire entre la paroi extérieure du premier compartiment et la paroi intérieure du réacteur pour la réception d'un volume de phase liquide.

15. Procédé selon l'une quelconque des revendications 1 à 14, la proportion d'hydrogène dans le gaz contenant de l'hydrogène étant d'au moins 95 pour cent.
